# EUROPEAN PATENT APPLICATION

(11) **EP 2 026 068 A1**
(43) Date of publication of application: **18.02.2009**
(21) Application number: 07114485.1
(22) Date of filing: 17.08.2007
(51) Int. Cl.: G01N 33/543, G01N 30/38

(54) **Method for producing purified and/or concentrated analytes, use and kit**

(71) Applicant: AOKIN AG, 13125 Berlin (DE)
(72) Inventor: Mallwitz, Frank, 12683 Berlin (DE); Levison, Derek, 10318 Berlin (DE); Dahmen-Levison, Ursula, 10318 Berlin (DE)
(74) Representative: Feldmann, Ute

(57) **Abstract**

The present invention relates to a method for producing purified and/or concentrated analytes of interest from a sample of biological sources, said method comprising or consisting of the following steps:
a) providing an affinity column capable of being centrifuged, the column comprising or consisting of:
(i) a centrifugation column,
(ii) optionally one, two or more separators, and
(iii) an affinity matrix comprising a matrix and one or more, the same or different binding moieties, wherein the binding moieties are attached to the matrix in such a way, that the binding moieties are capable of binding to the analytes of interest to form a binding moiety-analyte-complex so that the analytes of interest are not eluted from the column,

b) optionally washing the affinity column with a washing solvent,
c) adding the sample comprising the analytes of interest to and filtering the sample through the affinity column, so that at least part of the analytes of interest of the sample form the binding moiety-analyte-complex,
d) optionally washing the affinity column comprising the binding moiety-analyte-complexes with a washing solvent,
e) adding an incubating solvent to said affinity column comprising the binding moiety-analyte-complexes,
f) incubating the incubation solvent on the affinity column comprising the binding moiety-analyte-complexes, so that at least part of the binding moiety-analyte-complexes are destroyed and
g) eluting the analytes of interest from the affinity column by way centrifugation,
a use of an affinity column capable for being centrifuged, a kit comprising an affinity column and an affinity column.

## Description

The present invention relates to a method for producing purified and/or concentrated analytes from a sample of biological sources, a use of an affinity column capable for being centrifuged, a kit comprising an affinity column and an affinity column.

Analytes of interest are i.e. organic contaminants, in particular of environmental concern. For example the organic contaminant are fungal or microbial toxins (mycotoxins), which are fungal metabolites present in a large part of the world's food supply and pose a potential threat to food safety. Since their discovery, mycotoxins have attracted a great deal of interest and excellent research has been done by a number of scientific groups. In a further embodiment it can be of interest to purify analytes selected from the list of drugs, steroids, hormones, proteins, peptides, lipids, sugars, receptors, nucleic acids, vitamins, etc.

A range of highly sensitive and sophisticated analytical methods has been developed for the determination of analytes. Mostly chromatographic methods such as GC (Gas Chromatography) and HPLC (High Performance Liquid Chromatography) with a choice of different detectors are used. However, the determination step is usually preceded by a number of operations such as sampling, sample preparation, extraction and clean-up. The reliability of the results obtained by chromatography is highly dependent on the efficiency of these steps. The large number of components that are originally present in the sample must be reduced and interfering compounds that show the same behaviour in the chromatographic column must be removed as completely as possible.

Conventional techniques such as column chromatography and liquid-liquid extraction usually require high amounts of solvent (sometimes chlorinated), are time consuming, tedious to apply and a lot of experience is needed. Therefore, new approaches have been investigated to make extraction and clean-up procedures more easy to use.

A number of clean-up columns have been developed to be used after conventional extraction. They make use of different principles (immunoaffinity columns, solid phase extraction, ion exchangers). Less solvent is required and sample preparation can be speeded up considerably.

In WO 89/00293 a method for sample extraction, purification, concentration and quantification of low molecular weight organic compounds from biological sources, such as plant, is disclosed, wherein immunoaffinity columns are used. Immunoaffinity columns boast high selectivity as only the analyte is retained on the column by way of forming a specific antibody-analyte complex. In a rinsing step the interfering components can be rinsed off and finally the analyte can be eluted in a purified form from the column.

One drawback of this immunoaffinity column method refers to the fact that the analyte is eluted from the column with such an amount of solvent, so that it is necessary to conduct a further evaporating step before further analyzing the sample analyte. A further drawback of the state of the art refers to the fact that the purified analyte is often not provided in a concentrated form in comparison to the concentration in the sample to the purified.

Thus, one problem of the present invention relates to the provision of a method for producing purified analytes from samples of biological sources, which is less time consuming, less expensive and/or allows for concentration of the analyte.

The problem is solved by the subject matter as claimed in the independent claims.

Accordingly one aspect of the present invention relates to a method for producing purified and/or concentrated analytes of interest from a sample of biological sources, said method comprising or consisting of the following steps:
a) providing an affinity column capable of being centrifuged, the column comprising or consisting of:
   (i) a centrifugation column,
   (ii) optionally one, two or more separators, and
   (iii) an affinity matrix comprising a matrix and one or more, the same or different binding moieties, wherein the binding moieties are attached to the matrix in such a way, that the binding moieties are capable of binding to the analytes of interest to form a binding moiety-analyte-complex so that the analytes of interest are not eluted from the column,
b) optionally washing the affinity column with a washing solvent,
c) adding the sample comprising the analytes of interest to and filtering the sample through the affinity column, so that at least part of the analytes of interest form the binding moiety-analyte-complex,
d) optionally washing the affinity column comprising the binding moiety-analyte-complexes with a washing solvent,
e) adding an incubating solvent to the affinity column comprising the binding moiety-analyte-complexes,
f) incubating the incubation solvent on the affinity column comprising the binding moiety-analyte-complexes, so that at least part of the binding moiety-analyte-complexes are destroyed and
g) eluting the analytes of interest from the affinity column by way of centrifugation or low pressure.

Steps a) to g) of the inventive method can independently be conducted one or more times.

Accordingly a second aspect of the present invention relates to a use of an affinity column capable of being centrifuged, the column comprising or consisting of:
(i) a centrifugation column,
(ii) optionally one, two or more separators, and
(iii) an affinity matrix comprising a matrix and one or more, the same or different binding moieties, wherein the binding moieties are attached to the matrix in such a way, that the binding moieties are capable of binding to the analytes of interest to form a binding moiety-analyte-complex so that the analytes of interest are not eluted from the column,

for purification and/or concentration of analytes of interests in samples from biological sources.

A still further aspect of the present invention relates to a kit comprising or consisting of:
a) one or more affinity columns capable of being centrifuged, each column comprising or consisting of:
   (i) a centrifugation column,
   (ii) optionally one, two or more separators, and
   (iii) an affinity matrix comprising a matrix and one or more, the same or different binding moieties, wherein the binding moieties are attached to the matrix in such a way, that the binding moieties are capable of binding to the analytes of interest to form a binding moiety-analyte-complex so that the analytes of interest are not eluted from the column,
b) optionally one or more washing solvents for the affinity columns,
c) optionally one or more incubation solvents for incubating on the affinity columns comprising binding moiety-analyte-complexes, so that at least part of the binding moiety-analyte-complexes are destroyed, and
d) optionally one or more reagents for analyzing the analytes of interest.

A still further aspect of the present invention relates to an affinity column capable of being centrifuged comprising an opening where a sample is added and an opening where one or more analytes are eluted, characterized in that said column comprises an inner volume wherein the diameter of the inner volume is between 1 to 5 mm and the longitudinal length of the inner volume is between 10 to 30 mm.

The present invention is based on the finding that by using an incubation step, at least part of the binding moiety-analyte-complexes are destroyed on the affinity column, preferably immunoaffinity column, and thus, a higher percentage of free analytes of interests are present on the column before and during eluting the free analytes from the column. The incubation step is in particular preferred in connection with eluting the free analytes of interest by way of centrifugation or low pressure, as by way of centrifugation or low pressure, less time is available for destroying the binding moiety-analyte-complexes than by using conventional gravity forces. Preferably vaccum is applied as low pressure. And more preferably centrifugation forces are used. Accordingly, the present invention provides a method wherein less incubation solvent may be used to elute the analytes of interests from the column and less time is consumed for the purification and/or concentration process in comparison to conventional methods, where no incubation step and/or no centrifugation step is used. Further to this, the inventive method enables that the purified and/or concentrated analytes of interest are present in such a concentration so that the analytes may directly be analyzed in a further step without prior evaporating the solvent to reduce the amount of solvent as necessary when using methods of the state of the art.

In the meaning of the present invention "affinity chromatography" refers to a method of separating biochemical mixtures, based on the reversibility of highly specific biologic interactions such as that between antigen and antibody, enzyme and substrate, receptor and ligand, or protein-protein. Most commonly used components for the "affinity chromatography" are protein A, protein G and protein L. However, in the meaning of the present invention the term "immunoaffinity chromatography" is a subset of the broader term "affinity chromatography".

In the meaning of the present invention "immunoaffinity chromatography refers to a method of utilizing the specific interaction of an antigen-antibody reaction, where the antibody is immobilized onto a matrix and is capable of forming an antibody-analyte-complex so that the analyte cannot be washed off the column.

In the meaning of the present invention the term "wherein the binding moieties are attached to the matrix in such a way, that the binding moieties are capable of binding to the analytes of interest to form a binding moiety-analyte-complex so that the analytes of interest are not eluted from the column" means that a substantial amount of analytes of interest are not eluted from the column.

Preferred embodiments are disclosed in the following description and the dependent claims.

The present invention relates to the purification and/or concentration analytes of interests. In one embodiment the analytes of interest are of environmental or health concern. For example, the analyte of interest is a fungal or microbial toxin (mycotoxin).

Preferably the inventive method is being used for producing the same or different purified and/or concentrated mycotoxins selected from the group: aflatoxins, more preferably aflatoxins B1, B2, G1 and G2; ochratoxins, more preferably ochratoxin A (OTA), ochratoxin B, ochratoxin C, ochratoxin α and ochratoxin β, and most preferably ochratoxin A (OTA); type A-trichothecenes, more preferably T-2 toxin, HT-2 toxin, neosolaniol, monoacetoxy scirpenol and diacetoxyscirpe-nol; type B-trichothecenes, more preferably deoxynivalenol (DON), nivalenol, 3-and 15- acetoxynivalenol and fusarenon X; fumonisins, more preferably fumonisin B1 and fumonisin B2, and most preferably fumonisin B1; patulin; zearalenone; citrinin; cyclopiazonic acid; moniliformin; sterigmatocystin; alternaria toxins, more preferably tenuazonic acid, alternariol monomethyl ether, alternariol, altenuene and altertoxin I; ergot alkaloids, more preferably ergotamine, ergo-cornine, ergocristine, ergocryptine, agroclavine and ergometrine; *Aspergillus clavatus* toxins; *Aspergillus fumigatus* toxins ; citreoviridin; *Fusarium* toxins; gliotoxin; griseofulvin; mycophenolic acid; b-nitropropionic acid; kojic acid; penicillic acid; *penicilium* roqueforti-toxin (PR-Toxin), more preferably roquefortines A, B and C; penitrem A; stachybotryotoxin toxins, more preferably satratoxins G and H; viomellein, vioxanthin, xanthomegnin; and walleminols.

More preferably the inventive method is being used for producing the same or different purified and/or concentrated mycotoxins selected from the group: deoxynivalenol, zearalenone, and aflatoxin.

In another embodiment of the present invention, the analytes of interest are selected from - but not limited to - the group consisting of pesticides, drugs, steroids, hormones, proteins, peptides, lipids, sugars, receptors, nucleic acids, vitamins, etc. For example, in one embodiment, the analyte of interest is thyroxine, which is a major hormone secreted by the follicular cells of the thyroid gland.

The following Table 1 comprises analytes of interest:

| **CLASS** | **EXAMPLES** |
|---|---|
| pesticides | acetochlor and other acetanilides, atrazin, simazine, triazine, 2,4-D, 2,4,5-T, dichlorprop, MCPA, MCPB, triclopyr, pentachlorophenol, DDT, isoproturon, metha-benzthiazurone, metasulfuron-methyl, chlorsulfuron, propanil, paraquat, parathion-methyl, BTEX, nonylphenol, LAS |
| lipids | DHET |
| sugars | bacterial sugars, polysaccharides |
| peptides, proteins, receptors | IgG, albumin, receptors, KLH, LPH myoglobin, feto proteins, AT1 |
| oligonucleotides, DNA, RNA | of bacterial, animal, and/or plant origin, or specific sequences |
| toxins | zearalenone, deoxynivalenol, T-2, aflatoxins, ochratoxin, fumonisins, patulins, trichothecene, citrinin, cyclopiazonic acid, monoliformin, sterigmatocystin, alternaria-mycotoxins, ergot alkaloids |
| vitamins | vitamin B 12, vitamin C, folic acid |
| small molecules | chlorinated compounds, metals |
| therapeutic drugs, e.g., antiasmatics, antineoplastics, antiarythmics, anticonvulsants, antibiotics, antiarthritics, antidepressants, etc. | theophylline, doxorubicin, methotrexate, disopyramide, lidocaine, procainamide, propranolol, quinidine, N-acetyl-procainamide, Phenobarbital, phenotoin, pridon, valproic acid carbamazepine, ethosuximide, cephalosporins, erythromycin, tetracyclin, vancomycin, gentamicin, amikacin, chloramphenicol, streptomycin, tobramycin, penicillin, salicylate, nortriptyline, amitriptoline, imipramine, desipramine, histamine, thyroxine, triiodothyronine, serotonin, etc. |
| drugs of abuse | morphine, heroin, hydromorphone, dihydrocodeine, pholcodine, amphetamine, etc. |
| steroids and hormones | esterone, estradiiol, cortisol, testosterone, progesterone, chenodeoxycholic acid, digoxin, cholic acid, digitoxin, deoxycholic acid, lithocholic acid, etc. |
| prostaglandins | PGE, PGF, PGA, others |
| components of binding and reaction studies | antibodies, receptors, enzymes (i.e., proteases, nucleases, phosphatases) |

Preferably the analytes of interest purified and/or concentrated in the inventive method are of low molecular weight, in the meaning that preferably peptides, proteins, receptors are not analytes of interest. More preferably the analytes of interest are selected from the group of mycotoxins, lipids, vitamins, small molecules, small molecule therapeutic drugs and drugs of abuse, small molecule steroids and hormones, and small molecule components of binding and reaction studies, respectively including the preferred components. Still more preferably the analytes of interest are selected from the group of mycotoxins.

Thus, in a preferred method according to the present invention the same or different binding moieties of the affinity matrix are directed to bind specifically to the analytes of interests, including the preferred embodiments of the analytes of interest.

According to the present invention a sample comprising the analyte of interest is added to the centrifugation column. It may be the case that the sample first has to be prepared before adding the sample to the column.

In case mycotoxins are the analytes of interest an extraction from the source of origin, such as plants, according to standard methods may be conducted. Standard methods for the extraction of mycotoxins are disclosed, e.g. www.mycotoxins.org.

The following table 2 discloses conventional extraction methods for preferred mycotoxins as analytes of interests.

**Table 2:**

| | |
|---|---|
| aflatoxin | Extraction usually involves conventional procedures using acetone, chloroform and methanol or mixtures thereof. Small amounts of water give higher extraction efficiencies. Dunne et al. have described a multi-mycotoxin method, which uses hydrochloric acid and dichloromethane for extraction. |
| ochratoxin | Extraction is usually performed with a mixture of water and organic solvents, depending on the type of matrix. A IUPAC/AOAC method for the determination of OTA in barley uses a mixture of CHCl₃ and H₃PO₄ (1); for green coffee, CHCl₃ is only employed (3). For determination in wheat, a number of extraction solvents are used, including mixtures of toluene/HCl/MgCl₂, CHCl₃/ethanol/acetic acid and dichloromethane/H₃PO₄. |
| | There is a shift to non-chlorinated solvents, because of the environmental hazards involved. Burdaspal has proposed tert-butyl methyl ether as an extraction solvent for OTA in baby food (4). Other non-chlorinated solvent mixtures used for wheat samples include methanol/water and acetonitrile/water. Two methods for the determination of OTA in barley and roasted coffee have recently been validated: Extraction from barley was carried out with acetonitrile/water, and the extract was diluted with phosphate buffered saline solution (PBS) before a clean-up with immunoaffinity columns (5). |
| | A proficiency study for OTA in roasted coffee samples mainly employed a mixture of methanol with a 3% aqueous sodium bicarbonate solution (50:50) for extraction (6). |
| | Zimmerli and Dick (1995) have reported a method for the determination of OTA in red wine employing chloroform for extraction. |
| zearalenone | Solvents used for initial extraction are usually a mixture of various organic liquids including ethyl acetate, methanol, acetonitrile and chloroform. The use of chlorinated solvents is steadily decreasing, and acetonitrile in particular has become popular for extraction. |
| deoxynivalenol | Extraction of DON and other type B trichothecenes is performed by shaking or blending with aqueous acetonitrile. Also other solvents, such as water/PEG, and chloroform/methanol. Trenholm assessed extraction procedures for DON and could demonstrate that longer extraction times are required for naturally contaminated samples than for spiked ones. Extraction efficiency should therefore be evaluated with naturally contaminated samples. |

Samples from other biological sources than plants, may also be extracted by appropriate methods known in the art.

For the present invention conventional affinity columns capable of being centrifuged may be used. Usually affinity columns are used for capturing immunoglobulin G (IgG) or other antibodies. Usually mild eluting solvents are used, which destroy the binding moiety-analyte-complex, but do not change the tertiary structure of the proteins so that the binding properties etc of the proteins remain.

Companies providing affinity chromatography in spin column format are Pierce (Protein A on Agarose, Trisacryl or UltraLink; Protein A/G on Agarose or UltraLink; Protein G on Agarose or UltraLink; ProteinL on Agarose; Recomb Protein A on Agarose), Millipore, galab (AffinSpin-Kits).

Spin column format used for immunoprecipitation and co-immunoprecipitation refers to a purification procedure where it is determined whether two different molecules, usually proteins, interact. An antibody specific to the protein of interest is added to a cell lysis. Then the antibody-protein complex is pelleted usually using protein-G-sepharose which binds most antibodies. If there are any protein/molecules that bind to the first protein, they will also be pelleted. Identification of proteins in the pellet can be determined by western blot (if an antibody exists) or by sequencing a purified protein band.

AffinSpin-Kits are designed especially for the purification of microgram amounts of glycoproteins.

Spin columns for purification of DNA and RNA are manufactured by Epoch biolabs.

In a preferred embodiment immunoaffinity columns are used as affinity columns. In this preferred embodiment the columns comprise an immunoaffinity matrix, which comprises a matrix and as the binding moiety one or more, the same or different antibodies directed to specifically bind to the analytes of interest, in particular the preferred analytes of interest as set out above.

Preferred immunoaffinity columns are AHN Spin Columns (ahn biotechnology). Preferred are conical columns, such as Ion Exchange Spin Columns, i.e. from Pierce (Zeba Desalt Spin Columns), Clarification Spin Columns, i.e. from Agilent Technologies, Novagen, Primm Labs, QIAGEN, Sartorius AG, Sigma-Aldrich, Zymo Research, EPICENTRE, Invitrogen, Thermo Scientific and Millipore etc. (Filters, Spin, 0.22um, Cell Acet, pk/25; ClearSpin^{™} Filters, Easy Clean DNA Spin Filters, CORNING SPIN-X CEENTRIFUGE TUBE Filters, Zymo SpinOther Clarification Spin Columns, etc.).

Conventional immunoaffinity matrices may be used. For example the following matrixes can be used as immunoaffinity matrices: LiquiChip Activated Beads, in particular Epoxy-Activated Beads, Divinyl Sulfone (DVS) -Activated Beads and Divinyl Sulfone (DVS) - A - Activated Beads (i.e. from Qiagen or Adar Biotech), porous glass or beaded agarose.

In a further preferred embodiment of the inventive method one, two or more separators are used in the affinity column, and preferably the immunoaffinity column. Separators according to the present invention are capable of preventing that a substantial amount of the affinity matrix, preferably immunoaffinity matrix, gets washed off the column and more preferably additionally provide reproducible flow properties. Further to this a separator may be capable of preventing that a substantial amount of the affinity matrix, preferably immunoaffinity matrix, gets dispersed when e.g. the sample, the washing and/or incubating solvents are added to the column. Separators may be selected from - but not limited to - the group consisting of membranes; sintered polymers; frits, such as polymer frits, glass frits; glass wool; filters, such as glass fibres; minerals; papers; sand; charcoal; cotton; etc..

In case frits are used as preferred separators according to the invention, conventional frits from sintered polymer or glass may be used. More preferably the frit is made from sintered Polyethylen (PE). Still more preferably the frit provides a pore diameter which is small enough so that a substantial amount of the affinity matrix, preferably immunoaffinity matrix, does not get washed through the frit, and are big enough to provide reproducible flow properties. Thus, the pore diameter of a preferred frit according to the invention is preferably in the range of 20 to 70 µm.

In a preferred embodiment two separators are used in the affinity column, more preferably immunoaffinity column. In this case the second separator can also be selected from the group of separators as set out above. The second separator shall be capable of preventing that a substantial amount of the affinity matrix, more preferably the immunoaffinity matrix, gets dispersed when e.g. the sample, the washing solvents and /or the incubating solvents are added to the column comprising the matrix. In a still preferred embodiment a frit is used as the second separator. In a still preferred embodiment the frit of the second separator provides a pore diameter of 70 to 200 µm.

For the present invention conventional washing solvents may be used, which are mild and thus, not destroy at least substantial parts of the binding moiety-analyte-complexes, preferably the antibody-analyte-complexes. Usually such washing solvents can also be used as the loading solutions for the addition of the samples. Such solutions are preferably selected from the group consisting of buffers solutions, i.e. trishydroxymethylaminomethane (Tris), phosphate buffered saline (PBS), N-(2-hydroxyethyl)-piperazine-N'-2-ethanesulfonic acid (HEPES); deionized water and buffer with up to 10 and maximum up to 15 % organic solvents based on the weight of the whole buffer solution.

For the present invention incubation solvents may be used, which provide reversible or irreversible denaturing properties to the biding moiety-analyte-complexes, more preferably the antibody-analyte-complexes, and thus destroy at least part of the binding moiety-analyte-complexes, and more preferably the antibody-analyte complexes, so that the analytes of interest are set free and can be eluted in a further step from the column. Preferably the incubating solvents are selected from the group consisting of alcohols, more preferably methanol (MeOH) and isopropanol; solutions with a low pH, more preferably 100 mM glycine HCl with a pH of 2.5 to 3.0 or 100 mM citric acid with a pH of 3.0; solutions with a high pH, more preferably 50 to 100 mM triethylamine or triethanolamine with a pH of 11.5, 150 mM ammonium hydroxide with a pH of 10.5 and 0.1 M glycine NaOH with a pH 10.0; solutions with ionic strength, more preferably 2.0 to 5.0 M of lithium chloride, 2.0 to 5.0 M of magnesium chloride, 2.0 to 5.0 M of potassium chloride, 2.0 to 5.0 M of sodium iodide, 2.0 to 5.0 M of potassium iodide, 0.2 to 3.0 sodium thiocyanate and Tris-acetate with 2.0 M NaCl; denaturing solvents, more preferably guanidine, urea, ammonium thiocyanate, sodium deoxycholate and sodium dodecyl sulfate (SDS); organic solvents, more preferably dioxane and ethylene glycol; and competitor solutions, more preferably comprising counter ligands or analogs.

The incubating solvents are usually used in a range up to 5 ml, more preferably up to 3 ml, still more preferably up to 1 ml for one incubation step and subsequent elution of the analytes of interests of the column.

For the present invention the time for incubation of the incubating solvent in step e) of the inventive method is preferably up to 10 minutes, more preferably 0,5 to 6 minutes, and still more preferably 0,5 to 3 and still more preferably 2 minutes. In a further preferred method the incubation solvent is applied twice or more to the column and thus steps e), f) and g) of the inventive method are repeated twice or more times.

For the present invention a conventional apparatus can be used for centrifugation of the affinity column in the eluting step g). Such columns are defined according to the present invention also "packed" affinity column, which refers to the fact that the binding moieties, preferably antibodies, have formed a complex with the analytes of interest. Conventional apparatus for centrifugation comprise variable rounds per minutes (rpm) / g-factor.

A preferred method according to the present invention relates to a method, wherein the sample in step c) is also filtered through the affinity column, preferably immunoaffinity column, by way of centrifugation, or low pressure, preferably vacuum. Particularly preferred are centrifugation forces. This preferred inventive method is again less time consuming than the methods according to the state of the art, where the sample is filtered through by using gravity forces.

According to a further preferred method of the present invention a method is provided, wherein a reservoir is connected to said affinity column, preferably immunoaffinity column, and the sample in step c) is added in an amount using the reservoir. This means that the sample volume is larger than the volume provided by the affinity column, preferably the immunoaffinity column, alone, however, the sample volume is less or equal than the volume provided by the column and the reservoir together. Thus, by connecting the reservoir to the affinity column, preferably the immunoaffinity column, a larger volume of sample can be added to and filtered through the column. Thus, the purified analytes fraction may contain a higher concentration of analytes than using smaller amounts of the sample and accordingly a false negative result in a following analyzing step is less likely. A preferred volume of the reservoir is a volume of up to 20 ml.

In case the reservoir is connected to the column the sample is not filtered through the column by way of centrifugation, but by way of gravity or low pressure.

In case the reservoir is connected to the columns, a preferred affinity column, more preferably a preferred immunoaffinity column, is used for the present invention. This preferred column is adapted to being centrifuged. Further to this, said column provides an inner volume for use of the affinity matrix, preferably immunoaffinity matrix, of approximately 200 µl, preferably 50 to 100 µl and the diameter of the inner volume is between 1 to 5 mm, preferable 3 mm, and the longitudinal length of the inner volume is between 10 to 30 mm, preferably 20 mm. In particular preferred is an immunoaffinity column wherein the diameter of the inner volume is 3 mm and the longitudinal length of the inner volume is 20 mm. By using such a preferred column a volume approximately 50 to 100 µl immunoaffinity matrix is sufficient to achieve the same or better purification and/or concentration results according to the inventive method. Thus, a smaller volume of the cost intensive immunoaffinity matrix may be used for the inventive method whilst achieving the same or better purification and/or concentration results for the analytes, which further provides less costs for production of the column and thus carrying out the invention. As set out above the volume of the purified and concentrated analytes is such, that there is no evaporation step necessary for further analyzing the analytes.

The preferred embodiments already disclosed may also be used for the inventive use of the affinity columns, preferably immunoaffinity columns, the inventive kit or the inventive affinity column itself.

Said kit may optionally comprise one or more washing solvents, and one or more incubating solvents for the purification and/or concentration steps, and optionally reagents for analyzing the analytes of interest.

For the analytic step a competitive or direct reaction may be conducted. In the competitive reaction one or more labelled analytes in a known concentration are added (preferably in a cuvette, microtiter plate, etc.) to the purified and/or concentrated solution of analytes or interest. Further to this a binding moiety which specifically binds in a competitive reaction to the labelled analyte and the purified and/or concentrated analytes of interests (preferably antibodies) is also added in a known concentration. In the direct reaction one or more labelled binding moieties in a known concentration are added (preferably in a cuvette, microtiter plate, etc.) to the purified and/or concentrate solution of analytes of interest.

For the present invention conventional labelled analytes or binding moieties may be used for the analytic step. In case the label is a fluorescent moiety, then the fluorescent moiety can be selected from the (not exhaustive) list of 7-AAD, Acridine Orange, Alexa 488, Alexa 532, Alexa 546, Alexa 568, Alexa 594, Aminonapthalene, Benzoxadiazole, BODIPY 493/504, BODIPY 505/515, BODIPY 576/589, BODIPY FL, BODIPY TMR, BODIPY TR, Carboxytetramethylrhodamine, Cascade Blue, Coumarin,CY2, CY3, CY5, CY9, Dansyl Chloride, DAPI, Eosin, Erythrosin, Ethidium Homodimer II, Ethidium Bromide, Fluorescamine, Fluorescein, FTC, GFP (e.g. yellow shifted mutants T203Y, T203F, S65G/S72A), Hoechst 33242, Hoechst 33258, IAEDANS, Indopyras Dye, Lanthanide Chelate, Lanthanide Cryptate, Lissamine Rhodamie, Lucifer Yellow, MANT, MQAE, NBD, Oregon Green 488, Oregon Green 514,Oregon Green 500, Phycoerythrin, Porphyrin, Propidium Iodide, Pyrene, Pyrene Butyrate, Pyrene Maleimide, Pyridyloxazole, Rhodamine 123, Rhodamine 6G, Rhodamine Green, SPQ, Texas Red, TMRM, TOTO 1, TRITC, YOYO 1, Vitamin B12, flavin-adenine dinucleotide, 6-carboxy-X-rhodamine, nicotinamide-adenine, and dinucleotide. Preferably, the fluorescent conjugate would have a fluorescent wavelength different from competing fluorescent substances which may occur in host samples of interest, e.g., blood, serum, urine, tissue and extracts thereof.

In a preferred embodiment, the binding moiety that specifically binds to the labelled analyte and the analytes of interest is an antibody. It is well known that a substance, when injected into an animal, stimulates the animal to produce antibody. The antibody is capable of reacting with the injected substance in a highly specific manner. These antibodies belong to a group of serum proteins known as immunoglobulins. The production of these antibodies as a result of the injection of the antigen takes place over a period of many weeks, and depends upon the immunization schedule. In general, "good" antigens are usually of large molecular size (greater than 20,000 MW), partially digestible by enzymes and are recognized as being foreign by the antibody-producing animal.

In the following the present invention provides non-restrictive examples carrying out the invention.

### Figures:

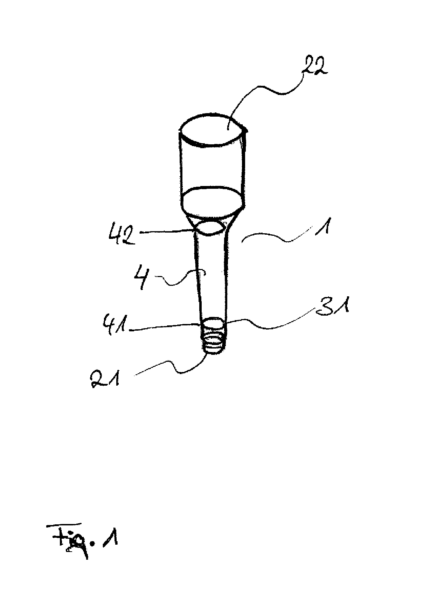

Figure 1 represents an inventive immunoaffinity column capable for being centrifuged.

### Detailed Description of Figures:

In Figure 1 an inventive affinity column, preferably immunoaffinity column 1 capable for being centrifuged is represented. The opening 21 represents the end of the column where the analytes are eluted. The opening 22 represents the end of the column where the sample and the solvents are added to the column. In use
the column 1 may optionally comprise a separator 31 placed in front of the opening 21, so that substantially no affinity matrix, preferably immunoaffinity matrix, (not shown) will be washed off the column during operation. Optionally column 1 further comprises a second separator 32 (not shown), which may be placed towards the second opening 22 in contact with the affinity matrix, preferably immunoaffinity matrix, so that the matrix (not shown) is not dispersed when the sample and solvents are added to the column. The volume between the distance from 41 to 42 refers to the inner volume 4, which comprises a diameter between 1 to 5 mm and a longitudinal length between 10 to 30 mm. Preferably for an immunoaffinity column the diameter of the inner volume is 3 mm and the longitudinal length is 20 mm. With such dimensions the use of 50 to 200 µl of the cost intensive immunoaffinity matrix is sufficient to achieve the same or even better purification and/or concentration results.

Further to this a reservoir (not shown) can be connected to the inventive immunoaffinity column 1 at the end of the opening 22.

### Example 1: Purification and / or concentration of sample with zearalenone mycotoxin

### 1. Loading of the column:

A frit (PE; pore size 35 µm) is placed in one end of the column, to prevent that the immunoaffinity matrix gets eluted off the column. On top of the frit 150 µl immunoaffinity matrix with an adequate concentration of the antibodies for zearalenone is applied, a second frit (PE, pore size 35 µm) is placed on top of the immunoaffinity matrix and the column is then sealed. For the column exemplary ZearaStarTM immunoaffinity columns (Romer) or other columns from Vicam or r-biopharm can be used.

The column has been brought to room temperature, then unsealed and the supernatant has been withdrawn by pipette.

### 2. Pre-washing step:

The column has been unsealed on the end of the column where the analytes are eluted off and placed in a reacting vessel of 2 ml. The column has then been washed with 800 µl of a PBS/MeOH - solution (PBS, ph 7.4, 90 vol.-% + MeOH 10 vol.-%) and centrifuged at 2000 rpm for 2 minutes.

### 3. Extraction and Sample Processing:

The extraction of the sample and the sample processing has been conducted according to the standard protocol (http://www.mycotoxins.org/) by using 15 g biological sample and 20 g extraction solvent (PBS/MeOH : water in the ration 80/20).

### 4. Loading of the column:

The extract is diluted by using PBS to a concentration of 1 part extract and 8 parts PBS. The amount of MeOH in the resulting solution is also 10 vol.-%.

800 µl of the diluted extract has been applied to the column and centrifuged at 2000 rpm for 2 minutes.

The analytes present in the extract are now bound to the antibodies present in the immunoaffinity matrix in the column ("packed column").

### 5. Washing step:

The packed column has again been washed by applying 800 µl of a PBS/MeOH solution (PBS 90 vol.-%/MeOH 10 vol.-%) to the column and has been centrifuged at 2000 rpm for 2 minutes.

### 6. Eluting step:

For eluting the analytes off the column, the column is placed in another reaction vessel.

150 µl MeOH has been applied to the column followed by an incubation step of 3 minutes. Thereafter the column is centrifuged by 2000 rpm for 2 minutes.

Further 150 µl MeOH has been applied to the column followed by a further incubating step of 3 minutes. Thereafter the column is centrifuged by 2000 rpm for 2 minutes.

### 7. Analytics

The eluate has been diluted with buffer solution so that the amount of MeOH is not higher than 10 vol.-%. Part of the eluate has been directly applied to a homogene immunoassay analysis for zearalene. No evaporation step was necessary.

### Example 2: Example 1: Purification and / or concentration of sample by using a reservoir

### 1. Loading of the column:

A frit (PE; pore size 35 µm) is placed in one end of the column, where the analytes will be eluted off. On top of the frit 150 µl immunoaffinity matrix with an adequate concentration of the antibodies for zearalenone is applied to, a second frit (PE, pore size 35 µm) is placed on top of the immunoaffinity matrix and the column is then sealed. For the column ZearaStarTM immunoaffinity columns (Romer) or other columns from Vicam or r-biopharm can be used.

The column has been brought to room temperature, then unsealed and the supernatant has been withdrawn by pipette.

### 2. Pre-washing step:

The column has further been unsealed on that end of the column where the analytes are eluted and placed in a reacting vessel of 2 ml. The column has then been washed with 800 µl of a PBS/MeOH - solution (PBS, ph 7.4, 90 vol.-% + MeOH 10 vol.-%) and centrifuged at 2000 rpm for 2 minutes.

### 3. Extraction and Sample Processing:

15 g of a biological sample has been extracted by using 20 g extraction solution MeOH 80 vol.% and PBS 20 vol.-%. The extract containing PBS has been filtered through a paper filter and has then been diluted so that the diluted solution only contained up to 10 vol.-% of PBS.

### 4. Connecting the column with the reservoir

The column then has been connected with the reservoir and protected against falling down.

### 5. Loading of the column:

20 ml of the extract has been applied to the reservoir. The extraxt is filtered through the column by way of gravitation (appr. 10 minutes).

Then the reservoir has been disconnected from the column.

The analytes present in the extract are now bound to the antibodies present in the immunoaffinity matrix in the column ("packed column").

### 6. Washing step:

The packed column has again been washed by applying 500 µl of a PBS/MeOH solution (PBS 90 vol.-%/MeOH 10 vol.-%) to the column and centrifuging at 1000 rpm for 2 minutes.

### 7. Eluting step:

For eluting the analytes off the column the column is placed in another reaction vessel.

100 µl MeOH has been applied to the column followed by an incubation step of 3 minutes. Thereafter the column is centrifuged at 1000 rpm for 2 minutes.

Thereafter further 100 µl MeOH has been applied to the column followed by and incubation step of 3 minutes. Thereafter the column is centrifuged at 1000 rpm for 2 minutes.

### 8. Analytics

Part of the eluate has been mixed with buffer and directly applied to a homogene immunoassay analysis. No evaporation step was necessary.

## Claims

1. A method for producing purified and/or concentrated analytes of interest from a sample of biological sources, said method comprising or consisting of the following steps:
a) providing an affinity column capable of being centrifuged, the column comprising or consisting of:
(i) a centrifugation column,
(ii) optionally one, two or more separators, and
(iii) an affinity matrix comprising a matrix and one or more, the same or different binding moieties, wherein the binding moieties are attached to the matrix in such a way, that the binding moieties are capable of binding to the analytes of interest to form a binding moiety-analyte-complex so that the analytes of interest are not eluted from the column,
b) optionally washing the affinity column with a washing solvent,
c) adding the sample comprising the analytes of interest to and filtering the sample through the affinity column, so that at least part of the analytes of interest form the binding moiety-analyte-complex,
d) optionally washing the affinity column comprising the binding moiety-analyte-complexes with a washing solvent,
e) adding an incubating solvent to said affinity column comprising the binding moiety-analyte-complexes,
f) incubating the incubation solvent on the affinity column comprising the binding moiety-analyte-complexes, so that at least part of the binding moiety-analyte-complexes are destroyed and
g) eluting the analytes of interest from the affinity column by way of centrifugation or low pressure.

2. Method according to claim 1, wherein the analytes are selected from the group of mycotoxins, pesticides, drugs, steroids, hormones, proteins, peptides, lipids, sugars, receptors, nucleic acids, vitamins.

3. Method according to claim 1 or 2, wherein the sample in step c) is filtered through the affinity column by way of centrifugation or low pressure.

4. Method according to claim 1 or 2, wherein a reservoir is connected to the affinity column and the sample in step c) is added in an amount using the volume of the reservoir.

5. Method according to claim 4, wherein said sample in step c) is filtered through by way of gravity or low pressure.

6. Method according to any one of claims, wherein the affinity column is an immunoaffinity column and the affinity matrix is an immunoaffinity matrix comprising a matrix and one or more, the same or different antibodies directed to specifically bind to the analytes of interest.

7. Use of an affinity column capable for being centrifuged, the column comprising or consisting of:
(i) a centrifugation column,
(ii) optionally one, two or more separators, and
(iii) an affinity matrix comprising a matrix and one or more, the same or different binding moieties, wherein the binding moieties are attached to the matrix in such a way, that the binding moieties are capable of binding to the analytes of interest to form a binding moiety-analyte-complex so that the analytes of interest are not eluted from the column,
for purification and/or concentration of analytes of interest from biological sources.

8. Kit comprising or consisting of:
a) one or more affinity columns capable for being centrifuged, each column comprising or consisting of:
(i) a centrifugation column,
(ii) optionally one, two or more separators, and
(iii) an affinity matrix comprising a matrix and one or more, the same or different binding moieties, wherein the binding moieties are attached to the matrix in such a way, that the binding moieties are capable of binding to the analytes of interest to form a binding moiety-analyte-complex so that the analytes of interest are not eluted from the column,
b) optionally one or more washing solvents for the affinity columns,
c) optionally one or more incubation solvents for incubating on the affinity columns comprising binding moiety-analyte-complexes, so that at least part of the binding moiety-analyte-complexes are destroyed, and
d) optionally one or more reagents for analyzing the analytes of interest.

9. Affinity column capable for being centrifuged comprising an opening where a sample is added and an opening where one or more analytes are eluted, **characterized in that** the column comprises an inner volume wherein the diameter of the inner volume is between 1 to 5 mm and the longitudinal length of the inner volume is between 10 to 30 mm.

10. Affinity column according to claim 9, **characterized in that** the column is an immunoaffinity column comprising an immunoaffinity matrix wherein the matrix comprises one or more, the same or different antibodies as binding moieties directed to specifically bind to the analytes of interest.
